# FASCICULE DE BREVET EUROPEEN

(11) **EP 2 519 267 B1**
(45) Date de publication et mention de la délivrance du brevet: **09.03.2016**
(21) Numéro de dépôt: 10798117.7
(22) Date de dépôt: 27.12.2010
(51) Int. Cl.: A61L 2/24, A61L 2/08, B65B 55/08, B67C 7/00, G21K 5/10, H01J 37/20

(54) **SYSTEME DE BLINDAGE EN FORME DE CLOCHE POUR DISPOSITIF DE TRAITEMENT DE RECIPIENTS PAR FAISCEAU D'ELECTRONS**
GLOCKENFÖRMIGES ABSCHIRMUNGSSYSTEM FÜR EINE VORRICHTUNG ZUR BEHANDLUNG VON ELEKTRONENSTRAHLENCONTAINERN
BELL-SHAPED SHIELDING SYSTEM FOR A DEVICE FOR TREATING ELECTRON-BEAM CONTAINERS

(30) Priorité: 29.12.2009 FR 0959655
(43) Date de publication de la demande: 07.11.2012
(73) Titulaire: Hema, 29000 Quimper (FR)
(72) Inventeur: LEJEUNE, Philippe, F-29000 Quimper (FR); MACQUET, Philippe, F-29000 Quimper (FR)
(74) Mandataire: Novagraaf Technologies
(86) Numéro de dépôt international: PCT/EP2010/070747
(87) Numéro de publication internationale: WO 2011/080245

(56) Documents cités:
- DE-A1- 4 407 183
- JP-A- 2003 121 597
- JP-A- 2004 069 456
- US-A- 4 944 132
- US-A1- 2008 073 549
- US-B2- 6 690 020

## Description

La présente invention concerne un dispositif de traitement pour la stérilisation de récipients par faisceau d'électrons, ledit dispositif étant équipé d'un système de protection particulier.

Il est connu, notamment par le document brevet US2009/0045350, des dispositifs de traitement comprenant un carrousel rotatif comportant un plateau support rotatif portant une pluralité de postes de traitement disposés à espace angulaire régulier, chaque poste de traitement comprenant des moyens de stérilisation qui comportent un émetteur de faisceau d'électrons, et des moyens de support pour supporter un récipient sous ledit émetteur, ledit émetteur étant apte à émettre un faisceau d'électrons passant par l'ouverture supérieure d'un récipient porté par lesdits moyens de support, afin de stériliser le récipient, en particulier la paroi interne dudit récipient.

Le document DE-A-4407183 décrit un dispositif pour la stérilisation de récipients en utilisant un émetteur UV. Par rapport à une stérilisation classique par voie chimique, la stérilisation par faisceau d'électrons se révèle plus efficace en termes de stérilisation, plus rapide, et ne laisse pas de traces résiduelles après traitement.

Les émetteurs de faisceaux d'électrons produisent toutefois des rayonnements indésirables, notamment des rayons X, et nécessitent donc de prévoir des systèmes de protection ou blindage pour éviter tout risque de propagation de rayonnements vers l'extérieur, et ainsi protéger les opérateurs. Les systèmes de protection sont formés par une enceinte de protection réalisée à base de plomb, dans laquelle est placé le carrousel rotatif pour la stérilisation, ainsi que les étoiles d'entrée et de sortie des récipients. De tels systèmes de protection s'avèrent lourds, encombrants, et très coûteux.

Le but de la présente invention est de proposer un dispositif de traitement pour la stérilisation de récipient avant remplissage, visant à pallier les inconvénients précités, qui soit performant et rapide, tout en restant simple de conception et de mise en oeuvre.

A cet effet, la présente invention propose un dispositif de traitement comprenant
- un ou plusieurs postes de traitement pour la stérilisation de récipients par faisceau d'électrons, chaque poste de traitement comprenant
- des moyens de stérilisation qui comportent un émetteur de faisceau d'électrons, et
- des moyens de support pour supporter un récipient sous ledit émetteur, ledit émetteur étant apte à émettre un faisceau d'électrons passant par l'ouverture supérieure d'un récipient porté par lesdits moyens de support afin de stériliser ledit récipient, en particulier la paroi interne du récipient, et
- un système de protection pour stopper les rayonnements émis par le ou les émetteurs,
   caractérisé en ce que ledit système de protection comprend, pour chaque poste de traitement,
- une partie supérieure assemblée à l'émetteur,
- une partie inférieure assemblée aux moyens de support, et
- des moyens de déplacement aptes à effectuer un déplacement relatif de la partie supérieure par rapport à la partie inférieure entre une position rétractée dans laquelle la partie supérieure est écartée de la partie inférieure, pour permettre le positionnement sous l'émetteur d'un récipient porté par les moyens de support, et au moins une position active dans laquelle la partie supérieure et la partie inférieure forment une enceinte de protection dans laquelle sont positionnés l'émetteur et le récipient porté par les moyens de support, afin d'effectuer les opérations de stérilisation.

Selon l'invention, la stérilisation est effectuée par faisceau d'électrons et le système de protection comprend, pour chaque émetteur une partie inférieure et une partie supérieure déplaçables l'une vers l'autre pour former une enceinte de protection, une même partie supérieure et/ou une même partie inférieure pouvant être communes à plusieurs émetteurs ou à tous les émetteurs du dispositif. Par rapport à des enceintes englobant l'ensemble du dispositif, l'enceinte de protection selon l'invention s'avère simple de conception et de réalisation, moins chère et moins encombrante.

Selon l'invention, ladite partie supérieure comprend une première platine ou premier plateau, dit supérieur, sur lequel est monté l'émetteur, ledit émetteur s'étendant vers le bas, la partie inférieure comprend une seconde platine, ou second plateau, dit inférieur, constituant lesdits moyens de support, la partie supérieure et la partie inférieure comprenant une jupe ou paroi tubulaire formant avec son plateau une cloche.

Selon l'invention, la partie supérieure se présente sous la forme d'une cloche formée du plateau supérieur et d'une paroi tubulaire, le plateau inférieur de la partie inférieure étant disposé dans la cloche lorsque la partie supérieure est dans une position active. Le plateau inférieur présente une paroi tubulaire s'étendant vers le bas, et apte à coulisser dans la paroi tubulaire de la partie supérieure, le chevauchement de ces parois tubulaires garantissant un blocage des rayonnements émis par le(s) émetteur(s).

Selon l'invention, ledit émetteur est équipé d'une buse tubulaire, s'étendant sensiblement verticalement sous le plateau supérieur et apte à délivrer par son extrémité distale le faisceau d'électrons créé par l'émetteur, ladite buse étant apte à s'introduire dans ledit récipient en passant par l'ouverture supérieure de ce dernier lors du déplacement de la partie supérieure vers la position active. Selon un mode de réalisation, lesdits moyens de déplacement sont aptes à déplacer la partie supérieure entre une première position active dans laquelle l'extrémité distale de la buse est disposée au dessus de l'ouverture supérieure du récipient et une position active extrême dans laquelle ladite buse s'étend dans le récipient.

Selon un autre mode de réalisation, l'une des parties du système de protection est sous la forme d'une cloche, ladite cloche venant sensiblement en appui par son bord libre contre le plateau de l'autre partie. Selon un autre mode de réalisation, la partie supérieure et la partie inférieure sont chacune formées d'une cloche, les deux cloches venant sensiblement en appui l'une contre l'autre dans une position active unique, ou de préférence s'emboîtent partiellement l'une dans l'autre dans des positions actives.

Selon un mode de réalisation, ledit dispositif est un dispositif de type linéaire, fonctionnant pas à pas, comprenant au moins une ou plusieurs voies ou lignes de traitement parallèles, par exemple de 1 à 12 lignes, de préférence de 6 à 12 lignes, chaque ligne étant équipée d'au moins un poste de traitement. Selon un mode de réalisation, le dispositif de traitement comprend
- plusieurs lignes parallèles entre elles,
- au moins une rangée de postes de traitement comprenant un poste de traitement pour chaque ligne de traitement,
- la partie supérieure du système de protection comprenant un plateau supérieur portant l'ensemble des émetteurs de ladite rangée de postes de traitement et une paroi tubulaire s'étendant vers le bas depuis ledit plateau supérieur et entourant l'ensemble desdits émetteurs de la rangée,
- la partie inférieure du système de protection comprenant un plateau inférieur, apte à porter au moins un récipient pour chaque ligne de traitement, constituant les moyens de support des postes de traitement de ladite rangée,
- lesdits moyens de déplacement étant aptes à déplacer ledit plateau supérieur et/ou le plateau inférieur, de préférence le plateau supérieur.

Le système de protection comprend une même cloche et un même plateau inférieur associé pour tous les postes de traitement de la rangée de postes de traitement.

Le dispositif peut comprendre au moins deux rangées successives de postes de traitement, parallèles entre elles. Dans ce cas, le système de protection comprend de préférence une partie supérieure, en particulier une cloche, et une partie inférieure pour chaque rangée de postes de traitement, de manière à former une enceinte de protection pour chaque rangée de postes de traitement.

Selon un autre mode de réalisation, le dispositif est de type rotatif, et comprend un carrousel comportant un plateau supérieur rotatif, portant une pluralité de postes de traitement disposés à espace angulaire régulier, le système de protection comprenant une partie supérieure et une partie inférieure pour chaque poste de traitement.

Avantageusement, les éléments constitutifs du système de protection sont réalisés à base de plomb.

La stérilisation par faisceau d'électrons peut générer une faible quantité d'ozone dans l'enceinte, en particulier dans le récipient placé dans l'enceinte. Selon un mode de réalisation, ledit système de protection comprend des moyens d'injection aptes à injecter un produit, dans l'enceinte, et notamment dans le récipient, pendant et/ou après l'irradiation par faisceau d'électrons, pour chasser l'ozone généré lors de l'irradiation par l'émetteur de faisceau d'électrons, et ainsi éviter toute détérioration par l'ozone du produit de remplissage qui sera conditionné dans le récipient. Le produit injecté est un produit gazeux ou un produit liquide à bas point d'ébullition, notamment un gaz neutre tel que de l'azote, ou de l'azote liquide. Avantageusement, lesdits moyens d'injection comprennent une canule d'injection, s'étendant de préférence le long de la buse de l'émetteur, ladite canule étant apte à s'introduire dans ledit récipient en passant par l'ouverture supérieure de ce dernier lors du déplacement de la partie supérieure vers la position active.

Selon un mode de réalisation, ledit système de protection, de préférence la partie supérieure du système de protection, et en particulier le plateau supérieur, comprend des moyens d'évacuation, pour l'évacuation vers l'extérieur de l'enceinte du gaz contenu dans l'enceinte, afin d'éviter toute surpression dans l'enceinte lors du déplacement relatif de la partie supérieure par rapport à la partie inférieure et/ou lors de l'injection d'un gaz neutre dans l'enceinte. Lesdits moyens d'évacuation comprennent par exemple un ou plusieurs trous d'évent, de préférence ménagés dans la paroi de la partie supérieure du système de protection, et notamment dans la partie supérieure du système de protection, chaque trou d'évent étant de préférence équipé d'une canalisation extérieure mise en dépression.

Selon un mode de réalisation, ledit système de protection comprend en outre un système de chicanes pour faire écran aux rayonnements émis par le ou les émetteurs à la liaison entre la partie supérieure et la partie inférieure, ledit système de chicanes comprenant par exemple un rebord annulaire de la paroi tubulaire inférieure, se prolongeant par une paroi annulaire, la paroi tubulaire supérieure s'insérant dans le logement formé entre la paroi annulaire et la paroi tubulaire inférieure lorsque la cloche est en position active.

L'invention sera mieux comprise, et d'autres buts, détails, caractéristiques et avantages apparaîtront plus clairement au cours de la description explicative détaillée qui va suivre d'un mode de réalisation particulier actuellement préféré de l'invention, en référence aux dessins schématiques annexés, sur lesquels :
- la figure 1 est une vue schématique de côté d'un dispositif de traitement selon l'invention comprenant un poste de traitement pour la stérilisation de récipients ;
- les figures 2A à 2C sont des vues de côté du poste de traitement illustrant les différentes positions du système de protection lors de la stérilisation d'un récipient ;
- la figure 3 est une vue schématique de dessus d'un dispositif de traitement selon une variante de réalisation, comprenant plusieurs postes de stérilisation en parallèle ;
- la figure 4 est une vue schématique selon le plan de coupe IV-IV de la figure 3 ;
- la figure 5 est une vue schématique selon le plan de coupe V-V de la figure 3 ; et
- la figure 6 est une vue schématique de côté d'un poste de traitement selon une variante de réalisation.

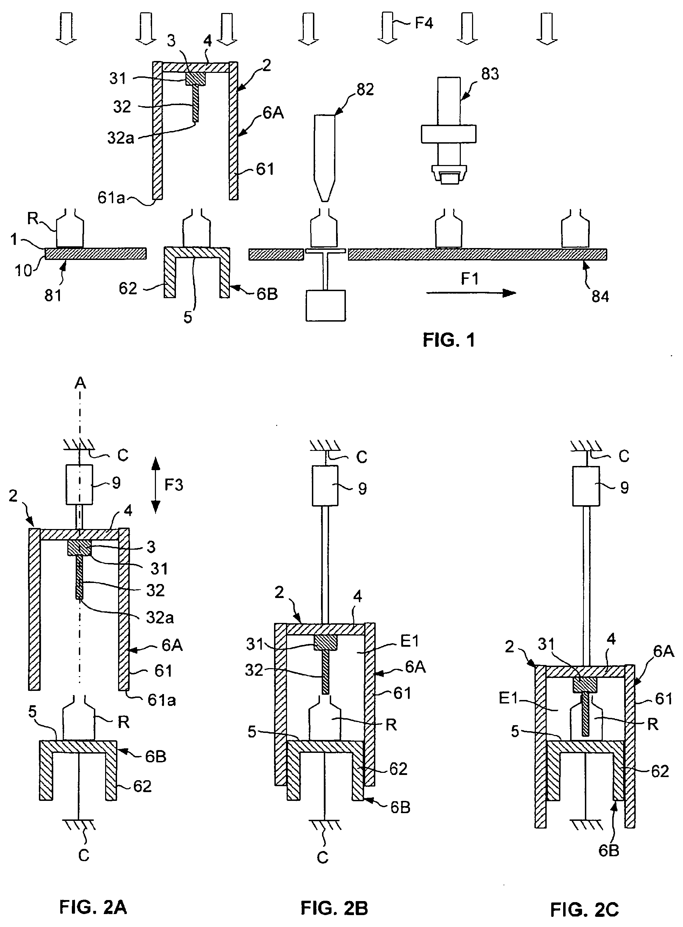

La figure 1 illustre un dispositif de traitement pour la stérilisation de récipients R, le remplissage des récipients traités avec un produit de remplissage et la fermeture des récipients.

Le dispositif de traitement comprend une ligne 1 de traitement comprenant un système de convoyage 10 fonctionnant pas à pas, le long duquel sont disposés d'amont en aval par rapport à la direction d'avancement F1, un poste 81 d'amenée de récipients, un poste 2 de traitement pour la stérilisation de récipients, un poste 82 de remplissage, un poste 83 de fermeture, et un poste 84 d'évacuation des récipients. Pour garantir un remplissage stérile des récipients, l'ensemble de la ligne de traitement est placé sous un flux laminaire d'air stérile, par exemple un flux laminaire descendant, tel que représenté schématiquement par les flèches F4.

Le dispositif selon l'invention permet la stérilisation, le remplissage et le bouchage de tout type de récipient R comprenant une ouverture principale supérieure. Le poste 2 de traitement permet la stérilisation de la paroi intérieure des récipients, en passant par l'ouverture supérieure des récipients. Le poste 82 de remplissage, connu en soi, par exemple de type pondéral, permet le remplissage des récipients stérilisés avec une quantité déterminée de produit de remplissage, le produit de remplissage étant liquide ou visqueux, par exemple un produit liquide tel que de l'eau, du lait ou du jus de fruit. Le poste 83 de fermeture ou poste de bouchage permet ici la mise en place d'un bouchon sur l'ouverture supérieure des récipients remplis.

Le poste 2 de traitement comprend des moyens 3 de stérilisation formés d'un émetteur 31 de faisceau à électrons, connu en soi, muni d'une buse tubulaire ou antenne 32 tubulaire, de forme allongée, l'émetteur étant apte à délivrer un faisceau d'électrons par l'extrémité 32a distale de sa buse. L'extrémité distale de la buse est équipée d'une feuille de titane. Les moyens de stérilisation sont par exemple formés d'un émetteur tel que décrit dans le document brevet US 2008/0073549. L'émetteur est monté fixe sur la face inférieure d'une platine ou plateau 4 dit supérieur, sa buse, d'axe A (Fig. 2A) longitudinal vertical, s'étendant verticalement vers le bas. La buse est définie de sorte qu'elle puisse être insérée par l'ouverture supérieure des récipients pour irradier l'intérieur des récipients. En référence à la figure 2A, la platine support est montée mobile sur le châssis C du dispositif, par l'intermédiaire de moyens 9 de déplacement, appelés également moyens de montée/descente, connus en soi, permettant de déplacer le plateau supérieur en translation verticale, tel qu'illustré par la flèche F3, entre une position rétractée haute, et des positions actives basses décrites ci-après.

Chaque poste de traitement comprend en outre des moyens de support d'un récipient R pour le maintien d'un récipient sous l'émetteur, sensiblement centré selon l'axe A de la buse. Les moyens de support comprennent une sellette ou plateau 5 dit inférieur, monté fixe sur le châssis C du dispositif, aptes à supporter un récipient. En variante, les moyens de déplacement agissent sur les moyens de support, la cloche supérieure étant montée fixe sur le châssis.

Le dispositif selon l'invention comprend un système de protection ou blindage pour stopper les rayonnements émis par les émetteurs, en particulier les rayonnements parasites du type rayons X. Ce système de blindage comprend une partie 6A supérieure et une partie 6B inférieure. La partie supérieure se présente sous la forme d'une cloche 6A formée par le plateau 4 supérieur et une paroi 61 tubulaire supérieure, appelée également jupe supérieure, s'étendant vers le bas depuis ledit plateau supérieur. Le plateau supérieur constitue la paroi de fond de la cloche, la jupe 61 supérieure entoure l'émetteur 31 et son antenne 32 et s'étend au-delà de l'extrémité distale 32a de l'antenne. La partie 6B inférieure est formée par ledit plateau 5 inférieur et par une paroi 62 tubulaire inférieure, appelée également jupe inférieure, s'étendant vers le bas depuis ledit plateau 5 inférieur. Le dispositif comprend par exemple une seule ligne 1 de traitement, avec un seul poste 2 de traitement pour la stérilisation. La cloche 6A de protection présente une forme générale cylindrique, son plateau 4 supérieur ayant la forme d'un disque, et sa jupe 61 supérieure ayant une section transversale circulaire. Le plateau 5 inférieur se présente également sous la forme d'un disque et sa jupe 62 inférieure présente une section circulaire dont le diamètre extérieur correspond sensiblement au diamètre intérieur de la jupe 61 supérieure.

En référence à la figure 2a, dans la position rétractée haute du plateau supérieur, le bord 61 a libre annulaire de la cloche 6A est disposé au dessus du plateau 5 inférieur, de sorte qu'un récipient R puisse être amené sur ledit plateau inférieur, sous l'émetteur et sous la cloche.

Pour l'opération de stérilisation d'un récipient, le plateau 4 supérieur est déplacé en translation vertical vers le bas, via les moyens 9 de déplacement, vers une première position active, illustrée à la figure 2B. Lors de ce déplacement, la cloche 6A vient recouvrir le récipient R, la jupe 62 inférieure de la partie inférieure coulissant dans la jupe 61 supérieure de la cloche. L'émetteur 31 et son antenne 32 sont disposés au dessus de l'ouverture du récipient. Le plateau 4 supérieur et la jupe 61 supérieure formant la cloche 6A, ainsi que le plateau 5 inférieur munie de sa jupe 62 inférieure, sont réalisés à base de plomb, et forment ensemble dans cette première position active une enceinte E1 de protection. Dans cette première position active, l'émetteur 31 peut être activé afin d'irradier la paroi externe du récipient. La jupe 61 supérieure et la jupe 62 inférieure se chevauchent sur une hauteur suffisante pour empêcher tout rayonnement de sortir de l'enceinte E.

Le plateau 4 supérieur est ensuite déplacé davantage vers le bas, via les moyens 9 de déplacement, jusqu'à la position active extrême illustrée à la figure 2C, afin d'irradier l'ensemble de la paroi interne du récipient. Lors de ce déplacement, la jupe 62 inférieure de la partie inférieure coulisse dans la jupe 61 supérieure de la cloche. La longueur de la buse 32 et la hauteur de la jupe 61 supérieure sont adaptées, de sorte que l'extrémité 32a distale de la buse soit disposée à proximité du fond du récipient R dans la position active extrême.

Le plateau 4 supérieur est alors ramené progressivement vers sa première position active basse illustrée à la figure 2B. L'irradiation est alors stoppée, et le plateau 4 supérieur est ramené dans sa position rétractée illustrée à la figure 2A. Le convoyeur 10 est alors activé pour évacuer le récipient traité et le transférer vers le poste 82 de remplissage, et pour amener un nouveau récipient à traiter sous l'émetteur.

Le dispositif selon l'invention peut être utilisé pour le remplissage stérile de différents récipients à un niveau de stérilisation de l'ordre de Log3.

Les récipients peuvent être de type bouteilles, tel qu'illustré schématiquement sur les figures, en verre ou en matériau plastique tel que PET, HDPE ou PP. Le remplissage est effectué au poste 82 de remplissage au moyen d'un bec de remplissage, et la bouteille est refermée, de préférence sous atmosphère saturée en azote, au poste 83 de bouchage qui comprend des moyens de vissage d'un bouchon. Selon un mode de réalisation, la bouteille est remplie au moyen de deux canules parallèles qui s'insèrent dans la bouteille et qui sont utilisées pour délivrer des produits différents. Une première canule est utilisée pour délivrer par exemple un liquide, tel qu'un jus de fruit, l'autre canule étant utilisée pour délivrer de la pulpe et/ou des vitamines par exemple.

Les récipients peuvent être des cartons du type «brique», classiquement formés d'un matériau complexe carton/LDPE /Aluminium. Les cartons sont préalablement préformés et transférés ouverts dans le dispositif. Les cartons sont ouverts au niveau de leur côté inférieur, ou au niveau de leur côté supérieur qui est éventuellement équipé d'une embase de capsule, thermo-soudée au matériau complexe et sur laquelle une capsule est vissée. Après stérilisation par insertion de l'antenne dans le récipient via son côté ouvert, les cartons sont remplis au poste de remplissage au moyen d'un bec de remplissage ou d'une ou deux canules, puis sont scellés au poste de fermeture, de préférence sous atmosphère saturée en azote, les moyens de vissage étant remplacés par un bloc de fers chauffants.

Les récipients peuvent être des cartons du type «Brique», préalablement préformés et déjà fermés par thermo soudure en partie haute, avec une embase de capsule, de 32 ou 36 mm par exemple, déjà thermo-soudée au matériau complexe. Les cartons sont transférés dans le dispositif avec l'embase ouverte, sans la capsule. Après stérilisation par insertion de l'antenne dans le récipient via l'embase ouverte, le remplissage est réalisé avec une canule, puis la fermeture est réalisée par vissage de la capsule sur l'embase.

Les récipients peuvent être des boîtes de conserve, en acier ou aluminium, qui sont serties après remplissage, le poste de fermeture comprenant alors un bloc sertissage.

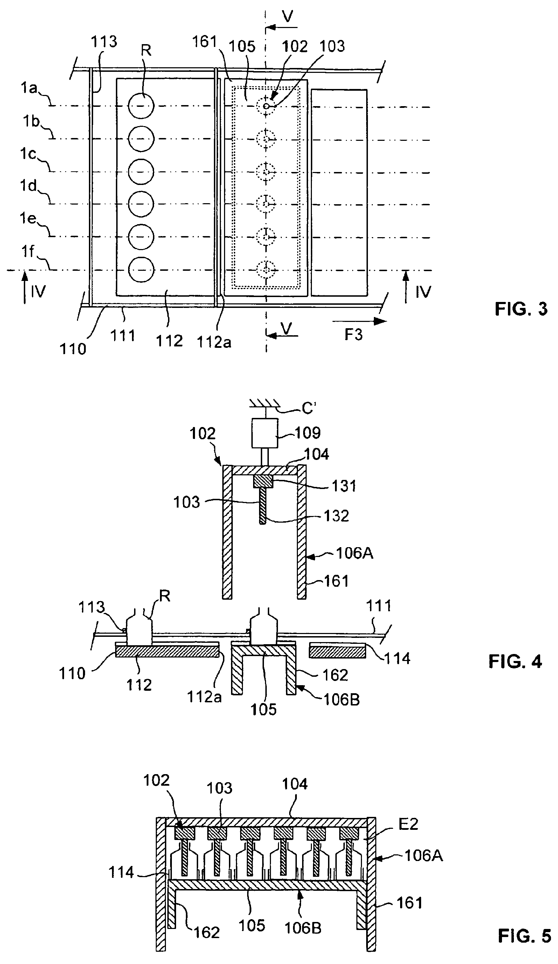

Les figures 3 à 5 illustrent une variante de réalisation dans laquelle le dispositif comprend 6 lignes 1a-1f de traitement parallèles, chaque ligne de traitement comprenant un poste 102 de traitement pour la stérilisation de récipients, les postes étant disposés selon une rangée perpendiculaire aux lignes de traitement.

Les émetteurs 131 sont montés sur la face inférieure d'un même plateau 104 supérieur, formé par exemple d'une plaque rectangulaire. Le plateau supérieur est monté mobile en translation sur le châssis C' du dispositif. Un plateau 105 inférieur commun, formé d'une plaque rectangulaire constitue les moyens de support de tous les postes 2 de traitement de la rangée de postes de traitement.

La partie supérieure du système de protection est formée d'une cloche 106A, constituée par ledit plateau 104 supérieur commun et une paroi tubulaire ou jupe 161 supérieure, de section rectangulaire. La partie 106B inférieure du système de protection est formée par ledit plateau 05 inférieur commun et une paroi tubulaire ou jupe 162 inférieure s'étendant vers le bas.

Des moyens 109 de déplacement (Fig.4), communs aux différents postes de traitement, permettent de déplacer le plateau 104 supérieur entre une position rétractée illustrée à la figure 4, et des positions actives, tel que décrit précédemment, et notamment une position active extrême illustrée à la figure 5, pour former une enceinte de protection E2 et permettre la stérilisation de six récipients simultanément.

L'entraînement des récipients R le long des 6 lignes de traitement est assuré par un convoyeur 110 à tasseaux, comprenant deux courroies 111 sans fin disposées de part et d'autre d'une sole 112 de transport, entre lesquelles sont montés des tasseaux 113. La sole 112 présente une ouverture 112a pour le positionnement du plateau inférieur et le passage de la paroi 162 tubulaire de la cloche.

Des rives 114 (Fig 5) longitudinales de guidage sont avantageusement prévues sur la sole et le plateau inférieur pour guider transversalement les récipients le long des lignes de traitement 1a-1f. En variante, le guidage transversal des récipients est assuré par les tasseaux, les tasseaux comprenant par exemple des empreintes dont la forme correspond à celle des récipients à convoyer.

Pour amener une rangée de récipients sur le plateau 105 inférieur, le plateau supérieur est amené en position rétractée haute, et le convoyeur est actionné pour déplacer les tasseaux dans la direction F3 et ainsi pousser une rangée de récipients sous les émetteurs, tel qu'illustré à la figure 4. Le convoyeur est ensuite actionné dans le sens inverse pour déplacer les tasseaux vers l'arrière et notamment écarter de l'ouverture 112a le tasseau ayant poussé les récipients sous les émetteurs afin de permettre le déplacement de la cloche 106A vers ses positions actives.

Chaque ligne du dispositif pourra également comprendre, en aval du poste de traitement 102, un poste de remplissage et un poste de bouchage tels que décrit précédemment.

En variante, chaque ligne 1a-1f de traitement comprend plusieurs postes de traitement disposés selon plusieurs rangées perpendiculaires aux lignes de traitement. Pour chaque rangée de postes de traitement, le système de protection comprend une cloche dont le plateau supérieur commun porte les émetteurs des postes de la rangée, et une partie inférieure dont le plateau inférieur constitue les moyens de support des postes de la rangée. Les cloches sont de préférence déplacées entre leur position rétractée et leur position active par des moyens 109 de déplacement communs. Les rangées de postes de traitement sont décalées les unes des autres afin de permettre le positionnement d'un tasseau entre deux cloches successives lorsque les cloches sont en position active.

Le système de protection selon l'invention peut également être utilisé avec des dispositifs de traitement de type rotatifs comprenant un carrousel comportant un plateau support ou plateau supérieur, sous la forme d'une plaque circulaire ou annulaire, destiné à être monté tournant sur un bâti fixe autour d'un axe de rotation vertical. Le plateau support porte une pluralité de postes de traitement disposés à espace angulaire régulier autour de l'axe de rotation. Chaque poste comprend un émetteur monté sur la face inférieure du plateau. La partie supérieure du système de protection est constituée par le plateau support et une pluralité de jupes dite supérieures entourant les émetteurs et s'étendant depuis la face inférieure du plateau. Chaque jupe supérieure forme avec le plateau supérieur une cloche associée à un poste de traitement. Les moyens de support de chaque poste de traitement comprennent une sellette ou plateau inférieur équipé d'une jupe inférieure. Chaque poste de traitement comprend des moyens de déplacement agissant cette fois-ci sur le plateau inférieur pour le déplacer entre une position écartée du plateau supérieur pour permettre le positionnement d'un récipient sur le plateau inférieur et le retrait d'un récipient du plateau inférieur, par exemple au moyen d'une étoile d'entrée et d'une étoile de sortie, et des positions actives dans lesquelles le plateau inférieur avec sa jupe supérieure forme avec la cloche supérieure une enceinte de protection.

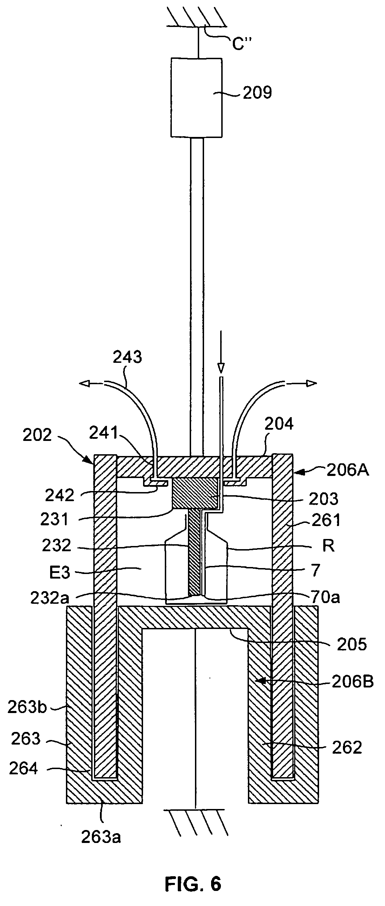

La figure 6 illustre une variante de réalisation d'un poste de traitement qui se différencie de celui illustré aux figures 2A à 2C par le fait qu'il comprend des moyens d'injection pour l'injection d'un produit dans l'enceinte, afin de chasser l'ozone généré lors de la stérilisation par faisceau d'électrons, et des moyens d'évacuation pour l'évacuation de gaz hors de l'enceinte. Le poste 202 de traitement comprend comme précédemment des moyens 203 de stérilisation comprenant un émetteur 231 de faisceau à électrons, muni d'une antenne 232 tubulaire, et monté sur un plateau 204 supérieur. Le système de protection comprend une cloche 206A supérieure formée par le plateau 204 supérieur et une paroi 261 tubulaire supérieure, et une partie 206B inférieure formée par un plateau 205 inférieur, muni une paroi 262 tubulaire inférieure. Le plateau supérieur est apte à être déplacé en translation verticale par des moyens 209 de déplacement, et le plateau inférieur, servant de support pour les récipients R, est monté fixe sur le châssis C".

Le poste de traitement comprend une tubulure ou canule 7 d'injection montée fixe dans la cloche. Cette canule s'étend parallèlement à l'antenne de l'émetteur, l'extrémité 70a distale de la canule étant disposée sensiblement au même niveau que l'extrémité 232a distale de l'antenne. Cette canule traverse de manière sensiblement étanche le plateau 204 supérieur de la cloche. La canule est utilisée pour injecter dans le récipient R un gaz neutre, tel que de l'azote, immédiatement après l'irradiation, par exemple lorsque le plateau 4 supérieur est dans sa première position active, afin de créer une surpression dans le récipient R et ainsi expulser hors du récipient l'ozone généré par l'irradiation. En variante, l'injection de gaz neutre est également réalisée lors de l'irradiation, par exemple lorsque le plateau supérieur est déplacé de sa position active extrême vers sa première position active.

En variante, la canule est utilisée pour délivrer dans le récipient R de l'azote liquide, par exemple une goutte d'azote liquide, pendant et/ou juste après l'irradiation, l'azote liquide passant en phase gazeuse remplit alors progressivement le récipient et chasse progressivement l'ozone hors du récipient.

Le plateau 204 supérieur est muni de trous 341 d'évent débouchant dans l'enceinte E3 pour éviter une surpression à l'intérieur de l'enceinte. Ces trous d'évents servent à évacuer le gaz qui est contenu dans l'enceinte et qui est comprimé lors de la descente du plateau 204 supérieur jusqu'à sa position active extrême. Par ailleurs, ces trous d'évent servent à évacuer l'ozone généré dans le récipient par l'irradiation et repoussé à l'extérieur du récipient R par l'azote injecté. Un système 242 de chicane, réalisé en plomb, est prévu sur la face inférieure du plateau supérieur pour faire écran aux rayons X générés lors de l'irradiation, et ainsi éviter toute propagation de rayonnement hors de l'enceinte par lesdits trous d'évent. Les trous d'évent sont connectés à des canalisations 243 flexibles, mises en dépression, afin d'aspirer le gaz comprimé par le déplacement relatif de la cloche et de la partie inférieure, et notamment l'ozone généré par l'irradiation.

Avantageusement, un système 263 de chicanes, réalisé également en plomb, est prévu pour éviter toute propagation de rayonnement au niveau de l'interface entre la partie 206A supérieure et la partie 206B inférieure, plus précisément à l'interface entre la paroi 261 tubulaire supérieure de la cloche, et la paroi 262 tubulaire inférieure. Le système 263 de chicanes comprend un rebord 263a annulaire horizontal s'étendant vers l'extérieur depuis le bord libre de la paroi 262 tubulaire inférieure, et se prolongeant par une paroi 263b annulaire verticale, qui s'étend vers le haut parallèlement à la paroi tubulaire inférieure, sensiblement jusqu'au plateau 205 inférieur. Cette paroi 263b annulaire forme avec la paroi 262 tubulaire inférieure un logement 264 annulaire dans lequel coulisse la paroi 261 tubulaire supérieure de la cloche lors du déplacement relatif de la cloche par rapport au plateau 205 inférieur.

Bien que l'invention ait été décrite en liaison avec un mode de réalisation particulier, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Dispositif de traitement comprenant
- un ou plusieurs de postes (2, 102, 202) de traitement pour la stérilisation de récipients (R) par faisceau d'électrons, chaque poste de traitement comprenant des moyens (3, 103, 203) de stérilisation qui comportent un émetteur (31, 131, 231) de faisceau d'électrons équipé d'une buse (32, 132, 232) tubulaire et des moyens (5,105, 205) de support pour supporter un récipient sous ledit émetteur, ledit émetteur étant apte à émettre un faisceau d'électrons passant par l'ouverture supérieure d'un récipient porté par lesdits moyens de support afin de stériliser ledit récipient, et
- un système de protection pour stopper les rayonnements émis par le ou les émetteurs, et comprenant pour chaque poste de traitement,
- une partie (6A, 106A, 206A) supérieure assemblée à l'émetteur et se présentant sous la forme d'une cloche formée d'un premier plateau (4, 104, 204) dit supérieur et d'une paroi (61, 161, 261) tubulaire s'étendant vers le bas depuis le plateau supérieure (4, 104, 204), ledit émetteur étant monté sur ledit premier plateau et sa buse s'étend sensiblement verticalement sous le plateau supérieur,
- une partie (6B, 106B, 206B) inférieure comprenant un second plateau (5, 105, 205), dit inférieur, constituant lesdits moyens de support,
**caractérisé en ce qu'**il comprend en outre des moyens (9, 109, 209) de déplacement aptes à effectuer un déplacement relatif de la partie supérieure par rapport à la partie inférieure entre une position rétractée dans laquelle la partie supérieure est écartée de la partie inférieure, pour permettre le positionnement sous l'émetteur d'un récipient porté par les moyens de support, et des positions actives dans lesquelles la partie supérieure et la partie inférieure forment une enceinte (E1, E2, E3) de protection ou blindage dans laquelle sont positionnés l'émetteur et le récipient porté par les moyens de support, le plateau inférieur étant disposé dans la cloche lorsque la partie supérieure est dans une position active, lesdits moyens de déplacement étant aptes à déplacer la partie supérieure entre une première position active dans laquelle l'extrémité distale (32a, 232a) de la buse (32, 132, 232) est disposée au-dessus de l'ouverture supérieure du récipient et une position active extrême dans laquelle ladite buse s'étend dans le récipient, le plateau (5, 105, 205) inférieur présentant une paroi (62, 162, 262) tubulaire s'étendant vers le bas depuis le plateau inférieur (5, 105, 205), apte à coulisser dans la paroi (61, 161, 162) tubulaire de ladite partie (6A, 106A, 206A) supérieure et permettant leur chevauchement.

2. Dispositif de traitement selon la revendication 1, **caractérisé en ce que** ledit dispositif est un dispositif de type linéaire, fonctionnant pas à pas, comprenant au moins une ou plusieurs lignes (1a-1f) de traitement parallèles, chaque ligne étant équipée d'au moins un poste (2, 102, 202) de traitement.

3. Dispositif de traitement selon la revendication 2, **caractérisé en ce qu'**il comprend
- plusieurs lignes (1a- 1f) parallèles entre elles,
- au moins une rangée de postes de traitement comprenant un poste de traitement pour chaque ligne de traitement,
- le plateau (4, 104, 204) supérieur de la cloche portant l'ensemble des émetteurs (31, 131, 231) de ladite rangée de postes de traitement et la paroi (61, 161, 261) tubulaire de la cloche s'étendant vers le bas depuis ledit plateau supérieur et entourant l'ensemble desdits émetteurs de la rangée,
- le plateau (5, 105, 205) inférieur constituant les moyens de support des postes de traitement de ladite rangée,
- lesdits moyens de déplacement étant aptes à déplacer ledit plateau supérieur et/ou le plateau inférieur.

4. Dispositif selon la revendication 1, **caractérisé en ce qu'**il est de type rotatif, ledit dispositif comprenant un carrousel comportant un plateau supérieur rotatif, portant une pluralité de postes de traitement disposés à espace angulaire régulier, le système de protection comprenant une partie supérieure et une partie inférieure pour chaque poste de traitement.

5. Dispositif de traitement selon l'une des revendications 1 à 4, **caractérisé en ce que** les éléments (4, 61, 5, 62 ; 104, 105, 161, 162 ; 204, 261, 205, 262) constitutifs du système de protection sont réalisés à base de plomb.

6. Dispositif de traitement selon l'une des revendications 1 à 5, **caractérisé en ce que** ledit système de protection comprend des moyens (7) d'injection aptes à injecter un produit dans l'enceinte (E3) pour chasser l'ozone généré lors de l'irradiation par l'émetteur (231) de faisceau d'électrons.

7. Dispositif de traitement selon la revendication 6, **caractérisé en ce que** lesdits moyens d'injection comprennent une canule (7) d'injection, ladite canule étant apte à s'introduire dans ledit récipient (R) en passant par l'ouverture supérieure de ce dernier lors du déplacement de la partie (206A) supérieure vers la position active.

8. Dispositif de traitement selon l'une des revendications 1 à 7, **caractérisé en ce que** ledit système de protection comprend des moyens (241, 242) d'évacuation, pour l'évacuation vers l'extérieur de l'enceinte (E3) du gaz contenu dans l'enceinte.

9. Dispositif de traitement selon l'une des revendications 1 à 8, **caractérisé en ce que** ledit système de protection comprend un système (263) de chicanes pour faire écran aux rayonnements émis par le ou les émetteurs à la liaison entre la partie supérieure (206A) et la partie inférieure (206B).

10. Dispositif de traitement selon la revendication 9, **caractérisé en ce que** ledit système de chicanes comprend un rebord (263a) annulaire de la paroi (262) tubulaire inférieure, se prolongeant par une paroi (263b) annulaire, la paroi (261) tubulaire supérieure s'insérant dans le logement (264) formé entre la paroi annulaire et la paroi tubulaire inférieure lorsque la cloche (206A) est en position active.

## Patentansprüche

1. Behandlungsvorrichtung, umfassend:
- eine oder mehrere Behandlungsstationen (2, 102, 202) zum Sterilisieren von Behältern (R) mit Elektronenstrahlen, wobei jede Behandlungsstation Sterilisierungsmittel (3, 103, 203) umfasst, die einen Sender (31, 131, 231) von Elektronenstrahlen umfassen, der mit einer röhrenförmigen Düse (32, 132, 232) und Tragmitteln (5, 105, 205) ausgestattet ist, um einen Behälter unter dem Sender zu tragen, wobei der Sender geeignet ist, einen Elektronenstrahl zu entsenden, der durch die obere Öffnung eines Behälters, der von den Tragmitteln getragen wird, hindurchgeht, um den Behälter zu sterilisieren, und
- ein Schutzsystem, um die von dem oder den Sendern entsandten Strahlungen zu stoppen, und umfassend für jede Behandlungsstation
- einen oberen Teil (6A, 106A, 206A), der mit dem Sender verbunden ist und in Form einer Glocke vorhanden ist, die von einer ersten so genannten oberen Platte (4, 104, 204), und einer röhrenförmigen Wand (61, 161, 261), die sich von der oberen Platte (4, 104, 204) nach unten erstreckt, gebildet ist, wobei der Sender auf der ersten Platte montiert ist und sich seine Düse im Wesentlichen vertikal unter der oberen Platte erstreckt,
- einen unteren Teil (6B, 106B, 206B), umfassend eine zweite so genannte untere Platte (5, 105, 205), die die Tragmittel darstellt,
**dadurch gekennzeichnet, dass** sie ferner Verschiebemittel (9, 109, 209) umfasst, die geeignet sind, eine relative Verschiebung des oberen Teils in Bezug zum unteren Teil zwischen einer eingezogenen Position, in der der obere Teil vom unteren Teil entfernt ist, um die Positionierung eines von den Tragmitteln getragenen Behälters unter dem Sender zu ermöglichen, und aktiven Positionen, in denen der obere Teil und der untere Teil einen Schutz- oder Abschirmungsraum (E1, E2, E3) bilden, in dem der Sender und der von den Tragmitteln getragene Behälter angeordnet sind, durchzuführen, wobei die untere Platte in der Glocke angeordnet ist, wenn der obere Teil in einer aktiven Position ist, wobei die Verschiebemittel geeignet sind, den oberen Teil zwischen einer ersten aktiven Position, in der das distale Ende (32a, 232a) der Düse (32, 132, 232) über der oberen Öffnung des Behälters angeordnet ist, und einer äußersten aktiven Position, in der sich die Düse in dem Behälter erstreckt, zu verschieben, wobei die untere Platte (5, 105, 205) eine röhrenförmige Wand (62, 162, 262) aufweist, die sich von der unteren Platte (5, 105, 205) nach unten erstreckt, die geeignet ist, in der röhrenförmigen Wand (61, 161, 162) des oberen Teils (6A, 106A, 206A) zu gleiten, und die ihre Überlappung ermöglicht.

2. Behandlungsvorrichtung nach Anspruch 1, **dadurch gekennzeichnet, dass** die Vorrichtung eine Vorrichtung linearen Typs ist, die schrittweise funktioniert, umfassend mindestens eine oder mehrere parallele Behandlungslinien (1a-1f), wobei jede Linie mit mindestens einer Behandlungsstation (2, 102, 202) ausgestattet ist.

3. Behandlungsvorrichtung nach Anspruch 2, **dadurch gekennzeichnet, dass** sie umfasst:
- mehrere zueinander parallele Linien (1a-1f),
- mindestens eine Reihe von Behandlungsstationen, umfassend eine Behandlungsstation für jede Behandlungslinie,
- wobei die obere Platte (4, 104, 204) der Glocke die Gesamtheit der Sender (31, 131, 231) der Reihe von Behandlungsstationen trägt, und wobei sich die röhrenförmige Wand (61, 161, 261) der Glocke von der oberen Platte nach unten erstreckt und die Gesamtheit der Sender der Reihe umgibt,
- wobei die untere Platte (5, 105, 205) die Tragmittel der Behandlungsstationen der Reihe darstellt,
- wobei die Verschiebemittel geeignet sind, die obere Platte und/oder die untere Platte zu verschieben.

4. Vorrichtung nach Anspruch 1, **dadurch gekennzeichnet, dass** sie drehenden Typs ist, wobei die Vorrichtung ein Karussell besitzt, umfassend eine drehende obere Platte, die eine Vielzahl von Behandlungsstationen trägt, die in einem regelmäßigen Winkelabstand angeordnet sind, wobei das Schutzsystem einen oberen Teil und einen unteren Teil für jede Behandlungsstation umfasst.

5. Behandlungsvorrichtung nach einem der Ansprüche 1 bis 4, **dadurch gekennzeichnet, dass** die Elemente (4, 61, 5, 62; 104, 105, 161, 162; 204, 261, 205, 262), aus denen das Schutzsystem besteht, auf Basis von Blei hergestellt sind.

6. Behandlungsvorrichtung nach einem der Ansprüche 1 bis 5, **dadurch gekennzeichnet, dass** das Schutzsystem Einspritzmittel (7) umfasst, die geeignet sind, ein Produkt in den Raum (E3) einzuspritzen, um das bei der Bestrahlung durch den Sender (231) von Elektronenstrahlen erzeugte Ozon zu vertreiben.

7. Behandlungsvorrichtung nach Anspruch 6, **dadurch gekennzeichnet, dass** die Einspritzmittel eine Einspritzkanüle (7) umfassen, wobei die Kanüle geeignet ist, sich in den Behälter (R) durch die obere Öffnung dieses letztgenannten bei der Verschiebung des oberen Teils (206A) in die aktive Position einzuführen.

8. Behandlungsvorrichtung nach einem der Ansprüche 1 bis 7, **dadurch gekennzeichnet, dass** das Schutzsystem Ableitungsmittel (241, 242) zur Ableitung des in dem Raum enthaltenen Gases aus dem Raum (E3) nach außen umfasst.

9. Behandlungsvorrichtung nach einem der Ansprüche 1 bis 8, **dadurch gekennzeichnet, dass** das Schutzsystem ein System (263) von Leitblechen umfasst, um eine Abschirmung gegen die von dem oder den Sendern entsandten Strahlungen an der Verbindung zwischen dem oberen Teil (206A) und dem unteren Teil (206B) zu bilden.

10. Behandlungsvorrichtung nach Anspruch 9, **dadurch gekennzeichnet, dass** das Leitblechsystem einen ringförmigen Rand (263a) der unteren röhrenförmigen Wand (262) umfasst, der durch eine ringförmige Wand (263b) verlängert wird, wobei sich die obere röhrenförmige Wand (261) in die Lagerung (264) einschiebt, die zwischen der ringförmigen Wand und der unteren röhrenförmigen Wand gebildet ist, wenn sich die Glocke (206A) in aktiver Position befindet.

## Claims

1. Processing device comprising
- one or several treatment stations (2, 102, 202) for the sterilisation of containers (R) via electron beams, each treatment station comprising sterilisation means (3, 103, 203) which comprise an electron-beam emitter (31, 131, 231) provided with a tubular nozzle (32, 132, 232) and supporting means (5, 105, 205) for supporting a container under said emitter, said emitter able to emit an electron beam passing through the upper opening of a container supported by said supporting means in order to sterilise said container, and
- a protection system in order to stop the radiation emitted by the emitter or emitters, and comprising for each treatment station,
- an upper portion (6A, 106A, 206A) assembled to the emitter and having the shape of a bell formed from a first plate (4, 104, 204) referred to as upper and from a tubular wall (61, 161, 261) extending downwards from said upper plate (4, 104, 204), said emitter being mounted on said first plate and its nozzle extends substantially vertically under the upper plate,
- a lower portion (6B, 106B, 206B) comprising a second plate (5, 105, 205), referred to as lower, constituting said supporting means,
**characterized in that** it further comprises moving means (9, 109, 209) able to carry out a relative movement of the upper portion in relation to the lower portion between a retracted position wherein the upper portion is separated from the lower portion, in order to allow for the positioning under the emitter of a container supported by the supporting means, and operative positions wherein the upper portion and the lower portion form a protective or shielding enclosure (E1, E2, E3) wherein are positioned the emitter and the container supported by the supporting means, the lower plate being arranged in the bell when the upper portion is in an active position, said moving means being able to move the upper portion between a first operative position wherein the distal end (32a, 232a) of the nozzle (32, 132, 232) is arranged above the upper opening of the container and an extreme operative position wherein said nozzle extends in the container, the lower plate (5, 105, 205) having a tubular wall (62, 162, 262) extending downwards from said lower plate (5, 105, 205), able to slide in the tubular wall (61, 161, 162) of said upper portion (6A, 106A, 206A) and enabling their overlapping.

2. Processing device according to claim 1, **characterised in that** said device is a device of the linear type, with step-by-step operation, comprising at least one or several parallel treatment lines (1a-1f), each line being provided with at least one treatment station (2, 102, 202).

3. Processing device according to claim 2, **characterised in that** it comprises
- several lines (1a-1f) parallel to each other,
- at least one row of treatment stations comprising a treatment station for each treatment line,
- the upper plate (4, 104, 204) of the bell supporting all of the emitters (31, 131, 231) of said row of treatment stations and the tubular wall (61, 161, 261) of the bell extending downwards from said upper plate and surrounding all of said emitters of the row,
- the lower plate (5, 105, 205) constituting the supporting means of the treatment stations of said row,
- said moving means able to move said upper plate and/or the lower plate.

4. Device according to claim 1, **characterised in that** it is of the rotating type, said device comprising a carrousel comprising an upper rotating plate, supporting a plurality of treatment stations arranged with regular angular spacing, the protection system comprising an upper portion and a lower portion for each treatment station.

5. Processing device according to one of claims 1 to 4, **characterised in that** the elements (4, 61, 5, 62; 104, 105, 161, 162; 204, 261, 205, 262) constituting the protection system are made from a lead base.

6. Processing device according to one of claims 1 to 5, **characterised in that** said protection system comprises injection means (7) able to inject a product into the enclosure (E3) in order to expel the ozone generated during the irradiation by the electron-beam emitter (231).

7. Processing device according to claim 6, **characterised in that** said injection means include an injection tube (7), said tube being able to be introduced into said container (R) by passing through the upper opening of the latter during the movement of the upper portion (206A) towards the active position.

8. Processing device according to one of claims 1 to 7, **characterised in that** said protection system comprises evacuation means (241, 242), for the evacuation towards the exterior of the enclosure (E3) of the gas contained in the enclosure.

9. Processing device according to one of claims 1 to 8, **characterised in that** said protection system comprises a system of baffles (263) in order to act as a screen to the radiation emitted by the emitter or emitters at the connection between the upper portion (206A) and the lower portion (206B).

10. Processing device according to claim 9, **characterised in that** said system of baffles comprises an annular edge (263a) of the lower tubular wall (262), extending via an annular wall (263b), the upper tubular wall (261) being inserted into the housing (264) formed between the annular wall and the lower tubular wall when the bell (206A) is in active position.
